# EUROPEAN PATENT APPLICATION

(11) **EP 1 876 548 A1**
(43) Date of publication of application: **09.01.2008**
(21) Application number: 06116757.3
(22) Date of filing: 06.07.2006
(51) Int. Cl.: G06F 19/00

(54) **Computational method for the prediction of enantiomeric excesses and absolute configurations by using DFT-based 3D descriptors**

(71) Applicant: Institut Catala D'Investigacio Quimica, 43007 Tarragona (ES)
(72) Inventor: Bo Jané, Carles, 43007, Tarragona (ES); Urbano Cuadrado, Manuel, 43007, Tarragona (ES); Carbó Martín, Jorge Juan, 43007, Tarragona (ES)
(74) Representative: Barlocci, Anna

(57) **Abstract**

A computer-based method for generating a three-dimensional structure activity relationship for a catalyst - substrate system comprising: (a) Generating 3D quantum descriptors by using a universal quantum mechanics based method; (b) Computing the electronic density and electrostatic potential; (c) Building a molecular isosurface; (c) Computing an steric descriptor at each isosurface 3D point; (d) Building predictive spaces by using structural aligning-free methods; (e) Combining the predictive spaces for the catalyst and the substrate; (f) Building a mathematical equation which matches combined predictive space according and chemical behaviour by using regression methods. Alternatively, a computer-based method for predicting the chemical behaviour of a catalyst - substrate system in a chemical process and a computer-based method for designing catalysts with a targeted chemical behaviour in a chemical process.

## Description

### Field of the Invention

The present invention relates to a computer-based method for predicting the chemical behaviour of a catalyst - substrate system, in particular the absolute configuration and the enantiomeric excess induced by said catalyst - substrate system in asymmetric catalytic transformations. The present invention is also directed to a method for the design of catalysts with a targeted chemical behaviour.

### Background Art

Enormous efforts are dedicated nowadays to obtain enantiomerically pure compounds to be used as building blocks in the synthesis of bioactive and pharmaceutical agents. Although most of the physical as well as chemical properties of enantiomers are very similar, chirality of biological molecules plays a key role in the activity of these compounds as drugs. Asymmetric homogeneous catalysis is becoming an important tool to obtain chiral compounds from raw materials. Most catalysts are transition metal complexes bearing chiral organic ligands. Therefore, there is a need to understand the role of the ligands in order to relieve ligand and catalyst design from trial-and-error approaches and make the performance of catalytic systems more predictable after small variations in the reactants and catalysts (Comprehensive Asymmetric Catalysis. E.N. Jacobsen, A. Pfaltz, H. Yamamoto (Eds.), Springer-Verlag, New York, 1999; P.W.N.M. Van Leeuwen, Homogeneous Catalysis, Kluwer Academic Publishers, Dordrecht, 2004).

The design of catalysts for inducing asymmetry can be undertaken with different methodologies. Aimed at developing predictive and modeling tools, Quantitative Structure-Selectivity Relationships (QSSR) approaches seek relationships between catalyst structures and their enantioselectivities. For this purpose, molecular descriptors which summarize pieces of chemical information are generated from 3D structures and related to enantioselectivity.

Comparative Molecular Field Analysis (CoMFA) (see R.D. III Cramer, D.E. Paterson, J.D. Bunce. Comparative Molecular Field Analysis (CoMFA). 1. Effect of Shape on Binding of Steroids to Carrier Proteins. J. Am. Chem. Soc. 110 5959-5967 (1988)) has become the most popular 3D-QSAR method because it describes, through grid generations, the steric and electronic roles played by each part of the system in the substrate-catalyst interaction. In addition, CoMFA and chirality are closely related QSAR areas since the 3D Molecular Interaction Field (MIF) values (the basis of the 3D grid descriptors) takes into account chirality by default. In this way, enantioselectivity of several families of catalysts has been studied by COMFA and COMFA-like methods (see K.B. Lipkowitz et al. Understanding Stereoiduction in Catalysis via Computer: New Tools for Asymmetric Synthesis. Synlett 125 1547-1565 (2003); K.B. Lipkowitz, M. Pradham. Computational Studies of Chiral Catalysts: A Comparative Molecular Field Analysis of an Asymmetric Diels-Alder Reaction with Catalysts Containing Bisoxazoline or Phosphinooxazoline Ligands. J. Org. Chem. 68 4648-4656 (2003); M.C. Kozlowski et al. Quantum Mechanical Models Correlating Structure with Selectivity: Predicting the Enantioselectivity of beta-Amino Alcohols Catalysts in Aldehyde Alkylation. J. Am. Chem. Soc. 125 6614-6615 (2003); J.L Melville et al. Computational Screening of Combinatorial Catalyst Libraries. Chem. Commun. 1410-1411 (2004); J. Huang et al. De Novo Chiral Amino Alcohols in Catalyzing Asymmetric Additions to Aryl Aldehydes. Org. Lett. 8 1565-1568 (2006)).

Other QSSR approaches based on constitutional, topological and geometrical descriptors (2D-QSSR) have been also developed (see A. Golbraikh et al. Novel Chirality Descriptors Derived from Molecular Topology. J. Chem. Inf. Comput. Sci. 41 147-158 (2001); A. Golbraikh, A. Tropsha. QSAR Modeling Using Chirality Descriptors Derived from Molecular Topology from Molecular Topology. J. Chem. Inf. Comput. Sci. 43 144-154 (2003); C. Jiang et al. QSAR Study of Asymmetric Reactions with Topological Indices. J. Chem. Inf. Comput. Sci. 43 1876-1881 (2003)). However, the design of new asymmetric catalysts can be guided in a better way by COMFA models: to evaluate what electronic or steric parts of the catalysts are important in enantiodiscrimination is straightforward.

Universal descriptors have not been employed in most of the CoMFA approaches. Thus, methods that make use of parameterization for the descriptor generation are involved in the development of QSSR models. Only a semiemprical quantum QSAR method has been developed by Dixon et al. (QMQSAR: Utilization of a Semiempirical Probe Potencial in a Field-Based QSAR Method. J. Comput. Chem. 2004, 26, 23-34). This method was the basis of the above cited QSSR model for studying the benzaldehyde ethylation using aminoalcohols.

### Summary of the Invention

The problem to be solved by the present invention is to provide a computer-based method for generating a three-dimensional structure - activity relationship, in particular a structure - enantioselectivity relationship, for a catalyst - substrate system, by using universal descriptors and thus avoiding parameterization and taking into account for the modelling both the catalyst and the substrate.

This problem is solved by the computer-based method for generating a three-dimensional structure activity relationship for a catalyst - substrate system according to a first aspect of the present invention, which comprises:
a. Generating 3D quantum descriptors for a series of training catalyst - substrate systems by using a universal quantum mechanics based method;
b. Computing the electronic density and electrostatic potential for a series of 3D points inside the grid which encloses each training catalyst - substrate system;
c. Building a molecular isosurface for each training catalyst - substrate system from the electronic density values computed in stage b;
d. Computing an steric descriptor at each isosurface 3D point;
e. Building predictive spaces for the catalyst and the substrate comprised in each training catalyst - substrate system from the electrostatic potential values computed in stage b and the steric descriptors computed in stage d by using structural aligning-free methods;
f. Combining the predictive spaces for the catalyst and the substrate built in stage e for each training catalyst - substrate system;
g. Building a mathematical equation which matches combined predictive space according to stage f and chemical behaviour in a given chemical process for each training catalyst - substrate system by using regression methods.

Advantageously, by using universal quantum mechanics based methods, no parameterization is employed, in other words, no empirical or semiempirical methods are used. As a consequence thereof, the method of the invention is able to represent any atom of the periodic table and therefore can be applied to any catalyst - substrate system and to any chemical process.

In a preferred embodiment of the present invention, said universal quantum mechanics based method is the *Density Functional Theory* (DFT). For example, ADF and Gaussian computational packages can be used.

Further advantages of employing quantum MIFs were considered, for instance the separation of nuclear and electronic effects, the inclusion of the delocalisation of the electron density across aromatic structures or the consideration of the non-spherical electron density of the p as well as d orbitals.

The optimal grid spacing to be considered according to the method of the present invention must be determined for each particular case. A medium-skilled person in the art will be able to find the optimal grid spacing for each system considered.

The calculation of the steric descriptor at each isosurface 3D point according to stage d of the method of the invention can be performed, for example, by using the coseno expression. MSD (Molecular Steric Descriptors) values are based on computing partial curvatures between the isosurface points and their neighbours. Convex and concave areas consist of isosurface points with [-1,0) and (0, +1] values, respectively.

Conveniently, after computing a steric descriptor at each isosurface 3D point according to stage d, the substantially most convex isosurface regions are selected, since these density regions are involved in stereochemical substrate-catalyst interactions with enantioselectivity influence.

Advantageously, the method according to the invention employs an alignment-free method so that descriptor spaces insensitive to grid orientation are built.

In a preferred embodiment of the present inventions, said alignment-free method is a GRIND-like method. The GRid-INdependent Descriptors (GRIND) method was described by Pastor et al. to avoid the alignment step involved in COMFA approaches (M. Pastor et al. GRid-INdependent Descriptors (GRIND): A Novel Class of Alignment-Independent Three-Dimensional Molecular Descriptors. J. Med. Chem. 43 3233-3243 (2000); F. Fontaine et al. Incorporating Molecular Shape into the Alignment-free GRid-INdependent Descriptors. J. Med. Chem. 47 2805-2815 (2004); F. Fontaine et al. Anchor-GRIND: Filling the Gap between Standard 3D QSAR and the GRid-INdependent Descriptors. J. Med. Chem. 48 2687-2694 (2005)). This step implies the positioning of key atoms of the training and test molecules at the same 3D positions according the *chiraphore* hypothesis. This fact requires *a priori* knowledge of the catalyst-substrate interaction and shows problems when very flexible structures are presented. The GRIND method has already been employed in catalysis (see S. Sciabola et al. Theoretical Prediction in Asymmetric Catalysis. An Alignment-Independent Molecular Interaction Field Based Approach. J. Org. Chem. 70 9025-9027 (2005)).

Advantageously, the method according to the present invention is that it considers both the catalyst and the substrate for the modelling, so that the prediction and design of the absolute configuration and enantiomeric excess for a given catalyst and for a given substrate can be carried out.

A suitable multivariate regression technique to be employed in stage g of the method of the invention is, for example, Partial Squares Least Regression (PLSR) (see S. Wold et al. PLS-Regression: A Basic Tool of Chemometrics, Chemom. Intell. Lab. Syst. 2001, 58, 109-130; P. Geladi. Partial Least Square Regression: A Tutorial. Anal. Chim. Acta 1985, 35, 1-17). PLSR involves both reducing the original data space and considering the variance of predictors and activities for the reduced space construction. PLSR offers the advantage of non-requiring predictive spaces with more objects than variables.

Conveniently, after building the the 3D structure - activity relationship according to the method of the present invention, a validation is performed in order to evaluate the accuracy, precision, robustness, etc., aimed at assuring the reliability of the predictive tool.

In a second aspect, the present invention relates to a computer-based method for predicting the chemical behaviour of a catalyst - substrate system in a chemical process, comprising:
a. Generating 3D quantum descriptors for a series of training catalyst - substrate systems by using a universal quantum mechanics based method;
b. Computing the electronic density and electrostatic potential for a series of 3D points inside the grid which encloses each training catalyst - substrate system;
c. Building a molecular isosurface for each training catalyst - substrate system from the electronic density values computed in stage b;
d. Computing an steric descriptor at each isosurface 3D point;
e. Building predictive spaces for the catalyst and the substrate comprised in each training catalyst - substrate system from the electrostatic potential values computed in stage b and the steric descriptors computed in stage d by using structural aligning-free methods;
f. Combining the predictive spaces for the catalyst and the substrate built in stage e for each training catalyst - substrate system;
g. Building a mathematical equation which matches combined predictive space according to stage f and chemical behaviour of each training catalyst - substrate system, by using regression methods;
h. Predicting the chemical behaviour of a catalyst - substrate system in a chemical process by using the mathematical equation built in stage g.

Surprisingly, the method for predicting the chemical behaviour of a catalyst - substrate system according to the present invention shows a good correlation with the corresponding experimental values. Moreover, the predicting methods according to the invention show good correlations for a broad structural diversity of both the catalyst and the substrate.

In a preferred embodiment, the method for predicting the chemical behaviour of a catalyst - substrate system according to the present invention is applied to the prediction of the absolute configuration or enantiomeric excess induced by a catalyst - substrate system in a asymmetric catalytic transformation. In a further preferred embodiment, the method of the invention can be applied to the prediction of both the absolute configuration and the enantiomeric excess induced by a catalyst - substrate system in a asymmetric catalytic transformation.

Advantageously, the method for predicting absolute configurations according to the present invention successfully predicts both *R*- and *S*- configurations. On the other hand, the method for predicting enantiomeric excesses of the invention successfully predicts enantiomeric excesses both when the R- configuration is in excess and when the *S*-configuration is in excess.

According to a third aspect, the present invention relates to a computer-based method for designing catalysts with a chemical targeted behaviour in a chemical process, comprising:
a. Generating 3D quantum descriptors for a series of training catalyst - substrate systems by using a universal quantum mechanics based method;
b. Computing the electronic density and electrostatic potential for a series of 3D points inside the grid which encloses each training catalyst - substrate system;
c. Building a molecular isosurface for each training catalyst - substrate system from the electronic density values computed in stage *b*;
d. Computing an steric descriptor at each isosurface 3D point;
e. Building predictive spaces for the catalyst and the substrate comprised in each training catalyst - substrate system from the electrostatic potential values computed in stage *b* and the steric descriptors computed in stage *d* by using structural aligning-free methods;
f. Combining the predictive spaces for the catalyst and the substrate built in stage e for each training catalyst - substrate system;
g. Building a mathematical equation which matches combined predictive space according to stage *f* and chemical behaviour in a given chemical process for each training catalyst - substrate system by using regression methods;
h. Analysing the relative influence of the catalyst and the substrate of test catalyst - substrate systems on the chemical behaviour by using the mathematical equation built in stage *g* and the predictive spaces;
i. Evaluating the relative influence of each functional group of the catalyst and the substrate on the chemical behaviour of each test catalyst - substrate system;
j. Using the information extracted from the stages *h* and *i* for designing catalysts with a targeted chemical behaviour.

Prior to a discussion of the detailed embodiments of the computer-based method according to the invention, a definition of specific terms related to the main aspects of the invention is provided.

A "three-dimensional" structure activity relationship as defined in the present invention refers to a mathematical equation which matches a series of variables providing molecular information (structural description) with the activity of a given compound. This equation can be a linear or a nonlinear (e.g. quadratic) function, or a non-parametric function.

A "training catalyst - substrate system" as defined in the present paper refers to the combination of a given catalyst and a given substrate whose structural description is an element of the data set employed for building (training) a "three-dimensional" structure activity relationship.

A "test catalyst - substrate system" as defined in the present paper refers to the combination of a given catalyst and a given substrate whose structural description of the catalyst - substrate system is an element of the data set employed for validating (testing) a "three-dimensional" structure activity relationship according to accuracy, precision, robustness, reversibility, etc.

The "chemical behaviour" as defined in the present invention refers to the outcome of a chemical process when using a catalyst - substrate system. Said chemical behaviour can be, for example, the enantiomeric excess induced, the absolute configuration induced, etc.

A "molecular isosurface" as defined in the present invention refers to the surface composed by the 3D points which show equal electronic density value. The molecular isosurface is employed to generate shape descriptors accounting for the steric characteristics of a molecule.

In a preferred embodiment of the computer-based method for designing catalysts according to the invention, the chemical targeted behaviour is selected from the group comprising absolute configuration, enantiomeric excess or the combination of both.

According to a preferred embodiment of the method of the invention, the catalyst is a metal catalyst which comprises a bidentate ligand.

According to another preferred embodiment of the method of the invention, the chemical process is selected from the group comprising alkylations, conjugated additions, aldol reactions and Mannich reactions.

Additional objects, advantages and features of the invention will become apparent to those skilled in the art upon examination of the description or may be learned by practice of the invention. The following examples and drawings are provided by way of illustration and are not intended to limit the scope of the invention. It is intended that the scope of the present invention be defined by the claims appended hereto.

### Brief description of the drawings

Figure 1 represents the sequential optimizations according to Example 1 for: (A) the number of isosurface points to be selected, and (B) the product distance rank employed in correlogram building.
Figure 2 show the equation coefficients (TIP-TIP and potential-potential coefficients) of the QSSR model built according to Example 1 for predicting e.e. and absolute configurations. Figure 2 (A) shows the coefficients for the catalyst subsystem and Figure 2 (B) the coefficients for the substrate substystem.
Figure 3 is a schematic representation of the optimized 3D structure and the selected isofurface points of the catalyst subsystem 67 of Example 1.

### Detailed description of particular examples

### Example 1. Prediction of the absolute configuration and enantiomeric excess in the aldehyde ethylation using Zn amino-alcohols as enantioselective catalysts

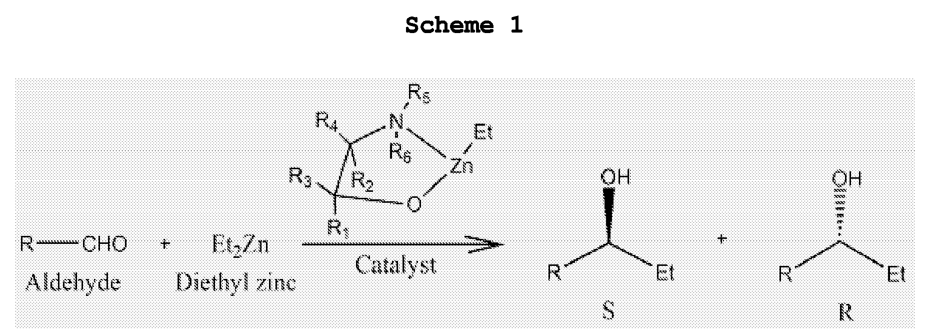

61 training catalyst - substrate systems were employed to construct a QSSR model according to the method of the invention. A large structural diversity was considered by means of varying the R₁₋₆ functional groups of the Zn-aminoalcohol catalyst shown in Scheme 1. Thus, aromatic and aliphatic substituents, small and bulky groups, rigid and flexible structures, cyclic and linear amines and so on were used. On other hand, different aliphatic and aromatic aldehydes were also considered.

In addition to the broad diversity in the aldehyde and aminoalcohol structures, a wide range of enantiomeric excess (e.e) values and both R and S configurations were considered.

### I. Quantum potential and electronic density distributions (step a + step b of the method according to the invention)

Quantum grids were generated for the optimized catalyst and aldehyde structures using the DFT methods implemented by ADF software. Electrostatic coulomb potentials were computed between the molecule under study and a proton probe placed at each one of the grid points. Electronic density values at all the grid points were also obtained.

The optimal grid spacing considered was 0.2 Å. In spite of being a small spacing compared to most of the CoMFA and CoMFA-like approaches, this choice is justified by the subsequent isosurface construction requiring a fine electronic density distribution. Grid dimensions also take into account a 5 Å distance beyond the 3D molecule positions.

### II. Calculation of 3D steric descriptors from electronic density distributions (step c + step d of the method according to the invention)

A molecular isosurface was generated from the electronic density distribution at a given density value • (0.002 a.u. in the present example).

The coseno expression was used to calculate curvature values at each of the grid points which form the isosurface, in a similar process to that shown by Pastor et al. in the background art (F. Fontaine, M. Pastor, F. Sanz. Incorporating Molecular Shape into the Alignment-free GRid-INdependent Descriptors. J. Med. Chem. 47 2805-2815 (2004)). TIP values are based on computing partial curvatures between the isosurface points and their neighbours. Convex and concave areas consist of isosurface points with [-1,0) and (0,+1] TIP values, respectively.

### III. Use of the GRIND methodology for constructing grid-independent predictive spaces (step e + step f of the method according to the invention)

The most relevant isosurface points were selected according to their TIP values -the most convex areas- and the MIF values were re-scaled. Afterwards, TIP-TIP and potential-potential auto-correlograms were constructed according to the method described by Pastor et al. and combined in order to obtain grid-independent predictive vectors.

Finally, the predictive vector (correlogram) was composed by a series of variables, each one representing a distance rank and showing the maximal MIF-MIF product per its distance rank. Since the XYZ coordinates of the points which produced the highest interactions were stored, reversibility was ensured.

### IV. Construction of the QSSR model/mathematical equation (step g of the method of the invention)

Partial Squares Least Regression (PLSR) was employed as the multivariate regression technique. PLSR involves both reducing the original data space and considering the variance of predictors and activities for the reduced space construction.

A predictive vector was composed by the sum of the TIP-TIP and potential-potential correlograms obtained for a given structure. In addition, when models for predicting e.e. of catalytic systems were developed, predictive vectors for the substrate and catalyst were also combined. Thus, the correlograms matrixes were composed by more variables than objects. PLSR offers the advantage of non-requiring predictive spaces with more objects than variables.

During the training and test stages, different statistical parameters were employed for evaluating the predictive ability of models, namely: correlation coefficient (r), standard error, slope and bias of the fitted/predicted vs. lab plots.

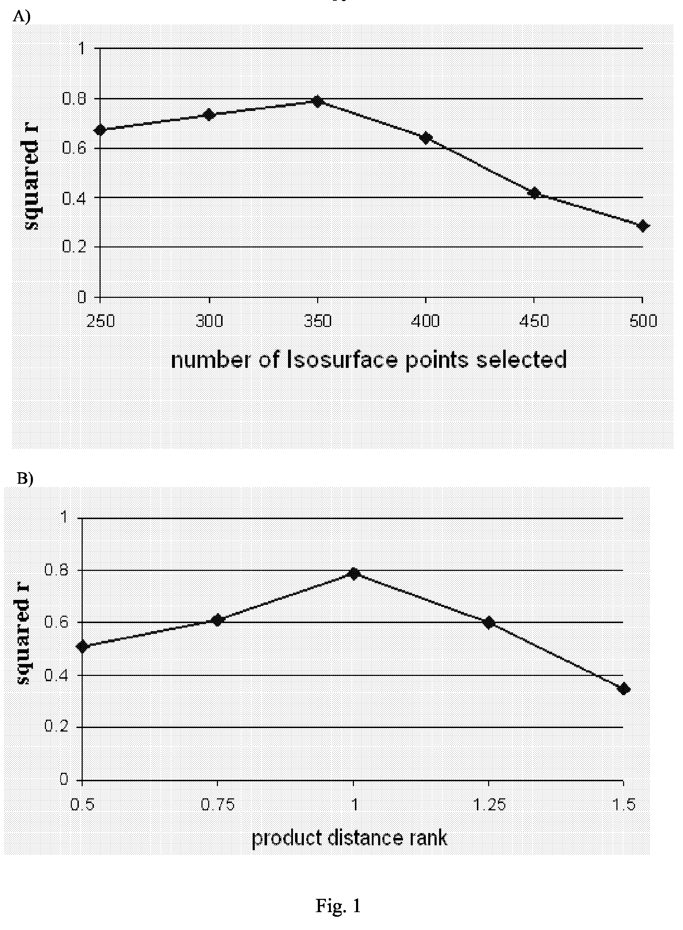

Sequential optimizations were carried out for the number of isosurface points to be selected and the product distance rank employed for constructing the correlograms. The r² obtained in prediction for full cross validations (FCV) was the optimization criterion. Variation of r² with the number of selected isosurface points was plotted at a distance rank = 1.00. The optimal value was found at 350, as can be seen in Figure 1 (A). A lower number would not permit to model important areas of big catalysts, while a higher number of selected points could add noise. When correlograms of substrates were built, the optimal number was found at 100 due to the smaller size of aldehydes considered.

Then, the distance rank was studied considering 350 points selected and its optimal value was found at 1 Å, as shown in Figure 1 (B). It is also important to remark that MIF products were computed from 3 up to 20 Å, ensuring this interval to include the key interactions without noise components.

The use of TIP-TIP and potential-potential auto-correlograms led to the better models and, so, TIP-potential cross-correlograms were discarded.

### V. Prediction of the absolute configuration and enantiomeric excess induced by several catalyst - substrate systems (step h according to the method of the invention)

Afterwards, the optimized model constructed in section IV was used to predict the absolute configuration and enantiomeric excess induced by several catalyst - substrate systems different from the training catalyst - substrate systems used in section I. The results are shown in Table 2.

The method for predicting the chemical behaviour of a catalyst - substrate system according to the present invention has been successfully applied to the prediction of the absolute configuration and enantiomeric excess induced in the ethylation of aldehydes catalyzed by Zn-aminoalcohol catalysts. The method has predicted successfully the absolute configuration of both *S*- and R-products, in contrast to the semiempirical method described in the background art (M.C. Kozlowski et al., Quantum Mechanical Models Correlating Structure with Selectivity: Predicting the Enantioselectivity of beta-Amino Alcohols Catalysts in Aldehyde Alkylation. J. Am. Chem. Soc. 125 6614-6615 (2003).), according to which only S products have been predicted.

Moreover, a good correlation between the predicted and experimental e.e. values has been found by the method of the present invention.

Advantageously, the predictive ability of the method according to the invention was maintained even by using structurally diverse catalysts and aldehydes (substrate) structures.

### VI. Evaluation of the relative importance of the different functional groups in the catalyst - substrate system

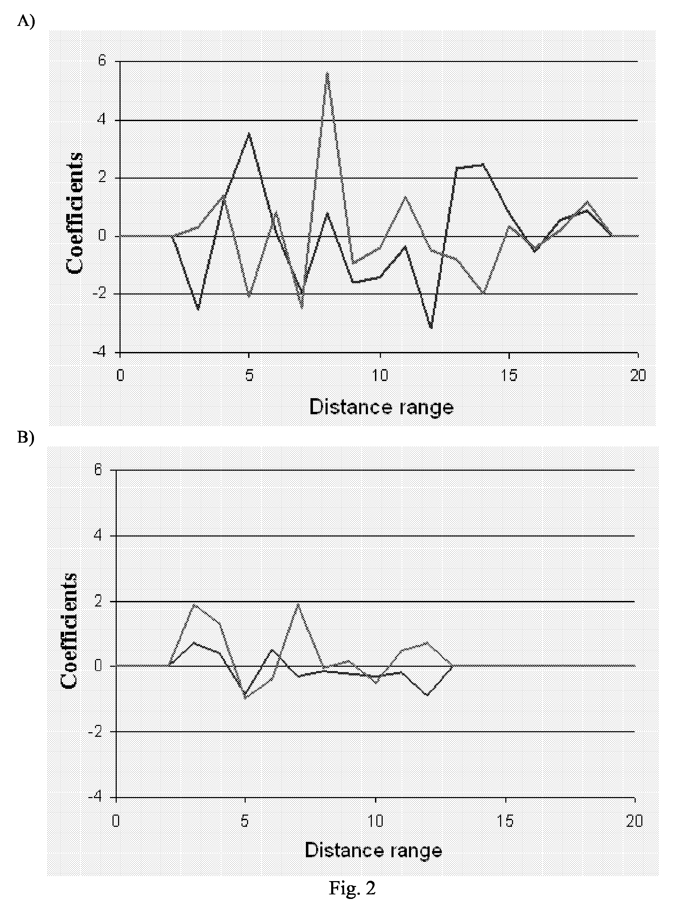

TIP-TIP (blue color) and potential-potential (green color) coefficients of the above described model for predicting R and S e.e. are shown in Figures 2(A) and (B) for the catalyst and substrate, respectively. As expected, the steric and electrostatic characteristics of catalysts had a greater effect than that of substrates in enantiodiscrimination.

Next, the relative influence of the different regions of the catalyst in the enantiodiscrimination achieved was evaluated according to their correlogram values weighted by the QSAR equation coefficients.

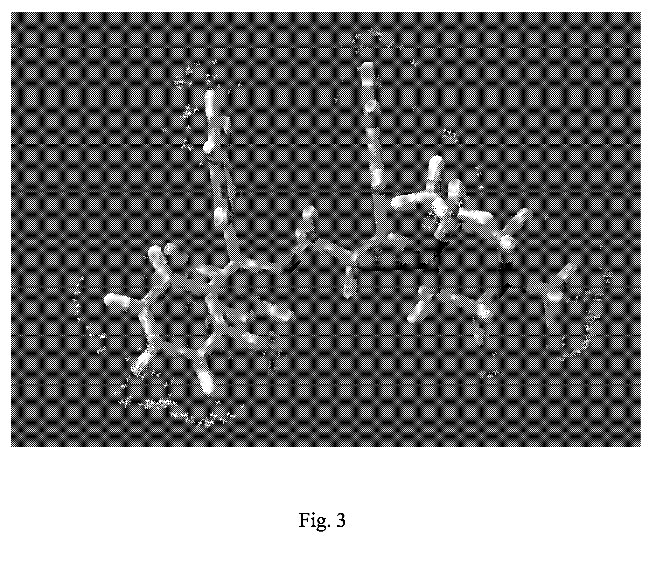

Figure 3 shows the catalyst structure of the system 67 and the isosurface points selected according TIP values. By substituent grouping of the points involved in the key interactions according the equation coefficients and the correlogram values was realized. Then, the contribution of each structural fragment was evaluated and expressed in relative terms.

Table 3 shows the functional groups of the catalyst core shown in Scheme 1 for four systems (62, 67, 71 and 72) and their influences in the alcohol configuration. As can be seen in the column corresponding to the global balance, the total influence is in agreement with the configuration obtained. For example, regarding the catalyst 62, the influence percentage of the regions which favour the S configuration is similar to that of the regions which address to the R alcohol (48 / 52 ratio). These similar influences agree with the fact of not having e.e. (3 %). Higher differences in the influence percentages are involved for the other catalysts. These influence gradients explain the experimental e.e. obtained.

This information can be used for designing catalysts which induce a target absolute configuration or e.e. according to the method of the present invention.

**Table 1. Training catalyst - substrate systems employed in Example 1**

| Cat -S system | -R₁ | -R₂ | -R₃ | -R₄ | -R₅ | -R₆ | Aldehyde | Conf. | e.e (%) | ΔG (kcal/mol) |
|---|---|---|---|---|---|---|---|---|---|---|
| 1 | -H | -H | -H | -H | -CH₂ | -CH₂ | 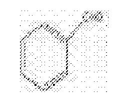 | - | 0 | 0.00 |
| 2 | -H | -H | 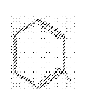 | -H | -CH₂ | -CH₂ | 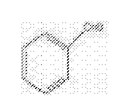 | S | 59 | 0.76 |
| 3 | -H | -H | 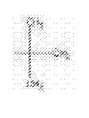 | -H | -CH₂ | -CH₂ | 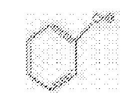 | S | 93 | 1.85 |
| 4 | -H | -H | -H | 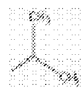 | -CH₂ | -CH₂ | 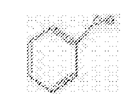 | S | 49 | 0.60 |
| 5 | -H | 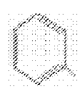 | 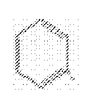 | -H | -CH₂ | -CH₂ | 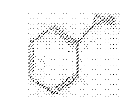 | S | 66 | 0.88 |
| 6 | -H | -H | 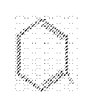 | 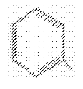 | -CH₂ | -CH₂ | 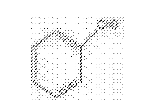 | S | 73 | 1.04 |
| 7 | -H | -H | 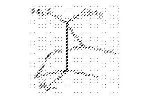 | | -CH₂ | -CH₂ | 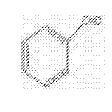 | S | 98 | 2.56 |
| 8 | -H | -H | 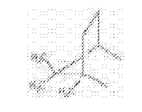 | | -CH₂ | -CH₂ | 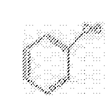 | S | 95 | 2.04 |
| 9 | -H | -H | 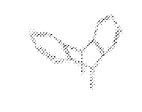 | | -CH₂ | -CH₂ | 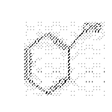 | S | 96 | 2.17 |
| 10 | -H | -H | 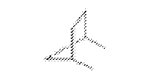 | | -CH₂ | -CH₂ | 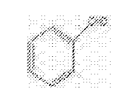 | S | 94 | 1.94 |
| 11 | -H | -H | 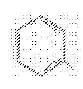 | -CH₂ | 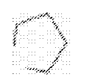 | | 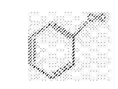 | S | 86 | 1.43 |
| 12 | 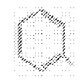 | -H | 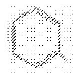 | 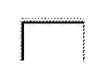 | | -CH₂ | 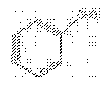 | S | 98 | 2.56 |
| 13 | -H | -H | 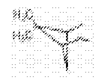 | | 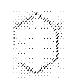 | | 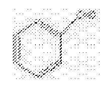 | S | 63 | 0.83 |
| 14 | -H | 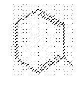 | 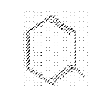 | 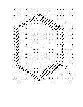 | -CH₂ | -CH₂ | 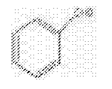 | S | 94 | 1.94 |
| 15 | -H | -H | 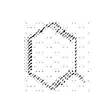 | 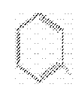 | 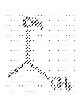 | 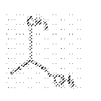 | 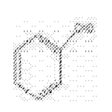 | S | 43 | 0.50 |
| 16 | -H | -H | 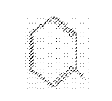 | 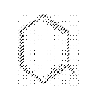 | 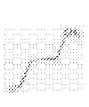 | 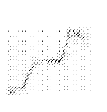 | 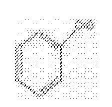 | S | 77 | 1.11 |
| 17 | -H | -H | 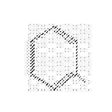 | 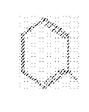 | 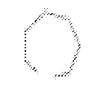 | | 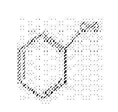 | S | 86 | 1.43 |
| 18 | -H | -H | 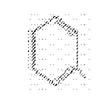 | 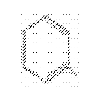 | 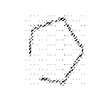 | | 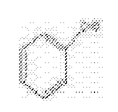 | S | 87 | 1.45 |
| 19 | -H | -H | 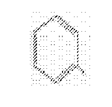 | 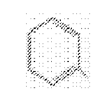 | 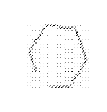 | | 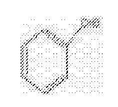 | S | 91 | 1.66 |
| 20 | -H | -H | 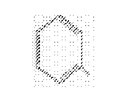 | 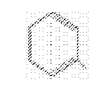 | 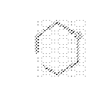 | | 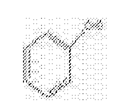 | S | 91 | 1.66 |
| 21 | -H | -H | 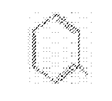 | 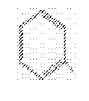 | 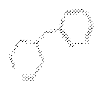 | | 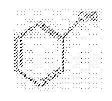 | S | 89 | 1.54 |
| 22 | -H | -H | 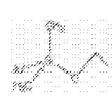 | 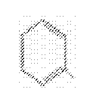 | 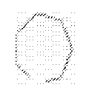 | | 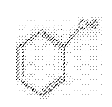 | S | 77 | 1.11 |
| 23 | -H | -H | 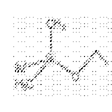 | 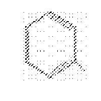 | 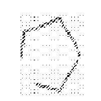 | | 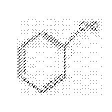 | S | 79 | 1.16 |
| 24 | -H | -H | 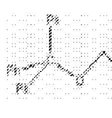 | 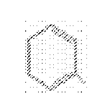 | -CH₂ | 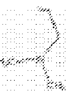 | 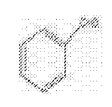 | S | 84 | 1.32 |
| 25 | -H | -H | 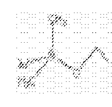 | 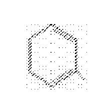 | 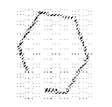 | | 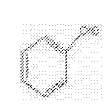 | S | 89 | 1.54 |
| 26 | -H | -H | 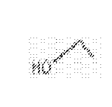 | 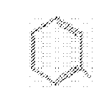 | 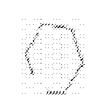 | | 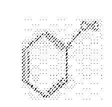 | S | 45 | 0.53 |
| 27 | -H | -H | 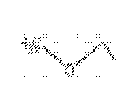 | 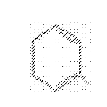 | 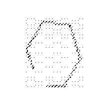 | | 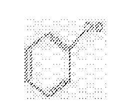 | S | 69 | 0.92 |
| 28 | -H | -H | 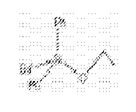 | 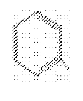 | 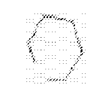 | | 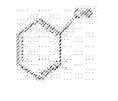 | S | 90 | 1.60 |
| 29 | -H | -H | 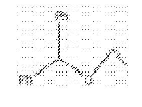 | 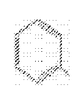 | 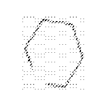 | | 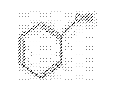 | S | 88 | 1.49 |
| 30 | -H | -H | 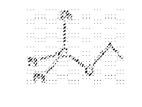 | 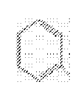 | 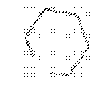 | | 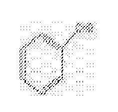 | S | 91 | 1.66 |
| 31 | -H | -H | 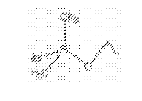 | 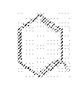 | 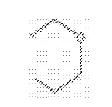 | | 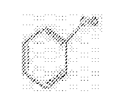 | S | 87 | 1.45 |
| 32 | -H | -H | 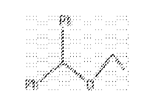 | 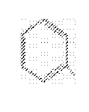 | 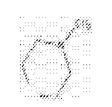 | | 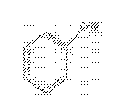 | S | 86 | 1.45 |
| 33 | -H | -H | 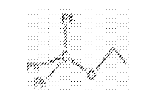 | 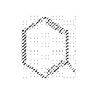 | 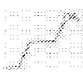 | 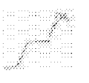 | 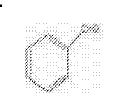 | S | 92 | 1.43 |
| 34 | -H | -H | 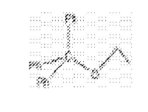 | 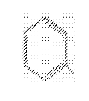 | 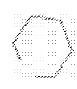 | | 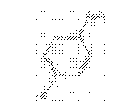 | S | 92 | 1.72 |
| 35 | -H | -H | 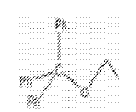 | 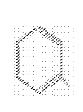 | 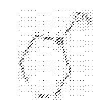 | 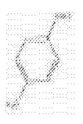 | S | 95 | 2.04 | |
| 36 | -H | -H | 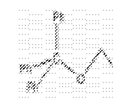 | 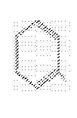 | 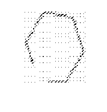 | 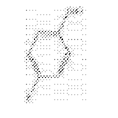 | S | 93 | 1.85 | |
| 37 | -H | -H | 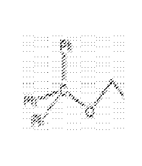 | 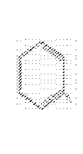 | 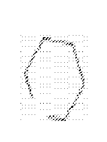 | 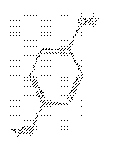 | S | 91 | 1.66 | |
| 38 | -H | -H | 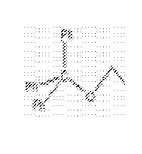 | 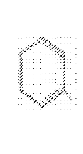 | 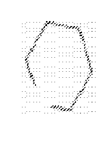 | 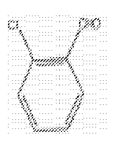 | S | 87 | 1.45 | |
| 39 | -H | -H | 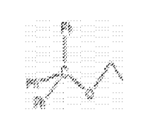 | 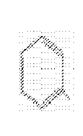 | 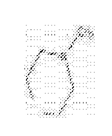 | 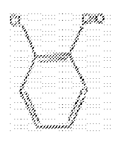 | S | 87 | 1.45 | |
| 40 | -H | -H | 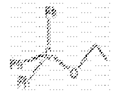 | 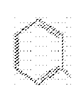 | 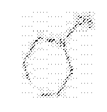 | | 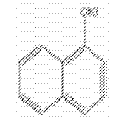 | S | 80 | 1.19 |
| 41 | -H | -H | 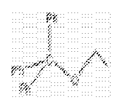 | 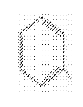 | 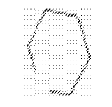 | | 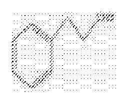 | S | 86 | 1.43 |
| 42 | -H | -H | 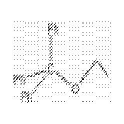 | 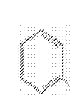 | 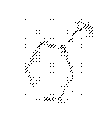 | | 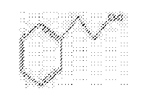 | S | 86 | 1.43 |
| 43 | -H | -H | 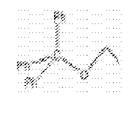 | 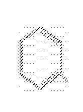 | 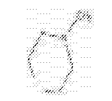 | | 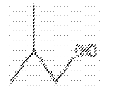 | S | 85 | 1.39 |
| 44 | -H | -H | 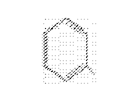 | 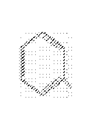 | 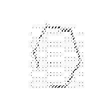 | | 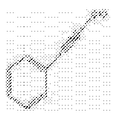 | S | 43 | 0.50 |
| 45 | -H | -H | 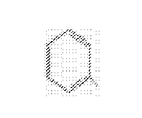 | -CH₂ | 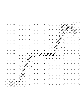 | 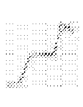 | 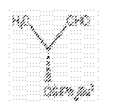 | S | 90 | 1.60 |
| 46 | 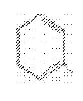 | -CH₂ | -H | -H | 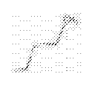 | 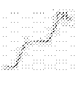 | 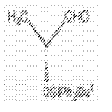 | R | 92 | -1.72 |
| 47 | -H | -H | 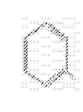 | -CH₂ | 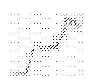 | 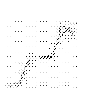 | 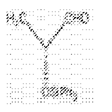 | S | 96 | 2.11 |
| 48 | 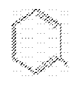 | -CH₂ | -H | -H | 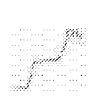 | 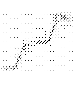 | 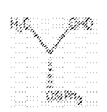 | R | 86 | -1.40 |
| 49 | -H | -H | 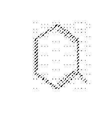 | -CH₂ | 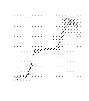 | 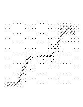 | 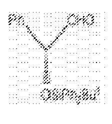 | S | 94 | 1.89 |
| 50 | -H | -H | 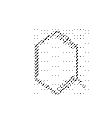 | -CH₂ | 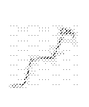 | 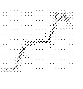 | 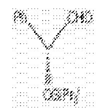 | S | 92 | 1.72 |
| 51 | -H | -H | 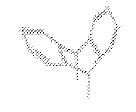 | | 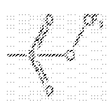 | -H | 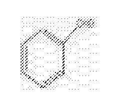 | R | 98 | -2.49 |
| 52 | -H | -H | 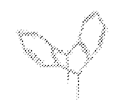 | | 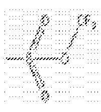 | -H | 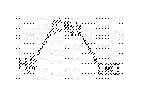 | R | 66 | -0.86 |
| 53 | -H | -H | 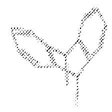 | | -CH₂ | -CH₂ | 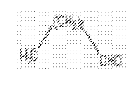 | S | 88 | 1.49 |
| 54 | -H | -H | 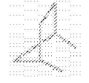 | | 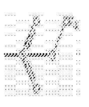 | -H | 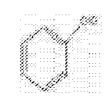 | S | 58 | 0.72 |
| 55 | 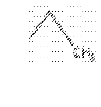 | -H | 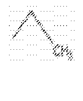 | 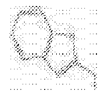 | -CH₂ | 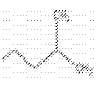 | 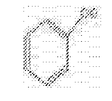 | R | 88 | -1.49 |
| 56 | 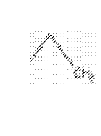 | -H | 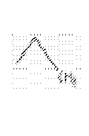 | 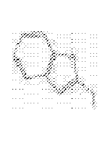 | -CH₂ | 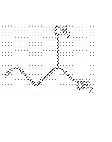 | 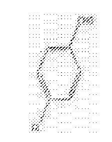 | R | 97 | -2.27 |
| 57 | 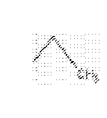 | -H | 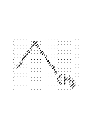 | 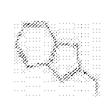 | -CH₂ | 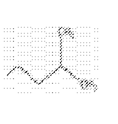 | 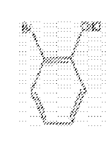 | R | 85 | -1.36 |
| 58 | 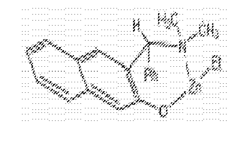 | | | | | | 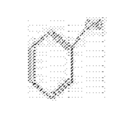 | R | 96 | -2.17 |
| 59 | 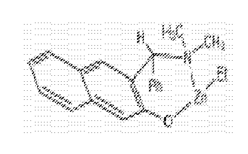 | | | | | | 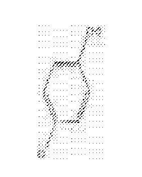 | R | 94 | -1.89 |
| 60 | 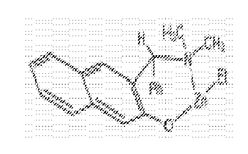 | | | | | | 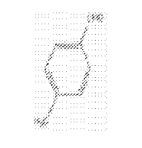 | R | 96 | -2.17 |
| 61 | 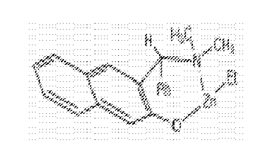 | | | | | | 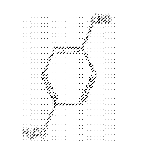 | R | 96 | -2.17 |

**Table 2. Experimental and predicted configurations and e.e. values**

| Cat. -S system | -R₁ | -R₂ | -R₃ | -R₄ | -R₅ | -R₆ | Aldehyde | Lab e.e. system (%) - Conf. | Predicte d e.e. (%) -Conf. |
|---|---|---|---|---|---|---|---|---|---|
| 62 | -CH₂ | -H | -H | -H | -CH₂ | - CH₂ | 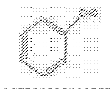 | 3 - S | 1 - S |
| 63 | -H | -H | 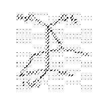 | | 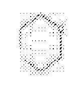 | | 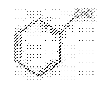 | 98 - S | 92 -S |
| 64 | -H | 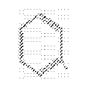 | 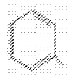 | 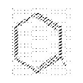 | 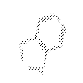 | | 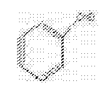 | 97 - S | 99 - S |
| 65 | -H | -H | 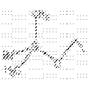 | 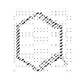 | 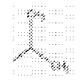 | | 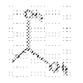 | 32 - S | 25 - S |
| 66 | -H | -H | 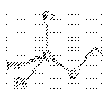 | 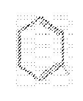 | 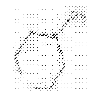 | | 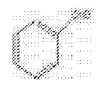 | 92 - S | 98 - S |
| 67 | -H | -H | 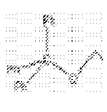 | 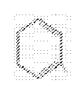 | 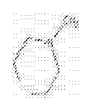 | | 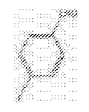 | 94 - S | 94 - S |
| 68 | -H | -H | 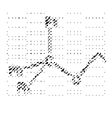 | 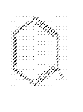 | 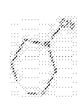 | | 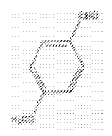 | 96 - S | 93 - S |
| 69 | -H | -H | 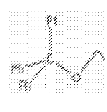 | 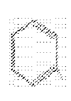 | 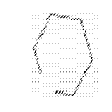 | | 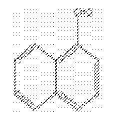 | 82 - S | 71 - S |
| 70 | -H | -H | 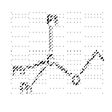 | 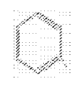 | 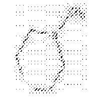 | | 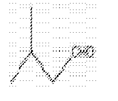 | 80 - S | 34 - S |
| 71 | 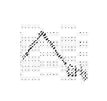 | -H | 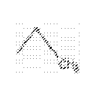 | 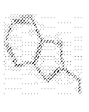 | -CH₂ | 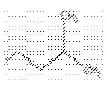 | 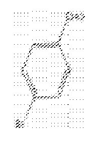 | 96 - R | 91 - R |
| 72 | 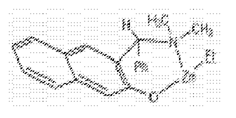 | | | | | | 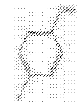 | 92 - R | 91 - R |

**Table 3. Influence of the different regions of the catalyst in the enant iodiscrimination**

| Cat. -S system | -R₁ | -R₂ | -R₃ | -R₄ | -R₅ | -R₆ | Ethyl | Global structural influence (%) in S / R | Lab e. e (%)-Conf |
|---|---|---|---|---|---|---|---|---|---|
| 62 | -CH₂ LOW influence in R | -H | -H | -H | -CH₂ Low influence in S | -CH₂ Low influence in S | Low influence in R | 48 / 52 | 3 - S |
| 67 | -H | -H | 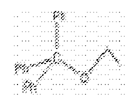 Low influence in S | 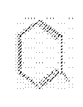 | 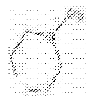 Low influence in R | | High influence in S | 63 / 37 | 94 - S |
| 71 | 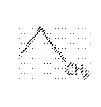 | -H | 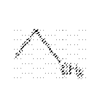 | 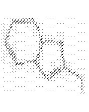 | -CH | 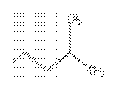 High influence in R | Low influence in S | 19 / 81 | 96 - R |
| 72 | 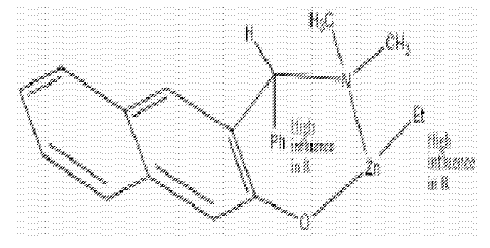 | | | | | | | 16 / 84 | 92 - R |

## Claims

1. A computer-based method for generating a three-dimensional structure activity relationship for a catalyst - substrate system comprising:
h. Generating 3D quantum descriptors for a series of training catalyst - substrate systems by using a universal quantum mechanics based method;
i. Computing the electronic density and electrostatic potential for a series of 3D points inside the grid which encloses each training catalyst - substrate system;
j. Building a molecular isosurface for each training catalyst - substrate system from the electronic density values computed in stage *b*;
k. Computing an steric descriptor at each isosurface 3D point;
l. Building predictive spaces for the catalyst and the substrate comprised in each training catalyst - substrate system from the electrostatic potential values computed in stage *b* and the steric descriptors computed in stage *d* by using structural aligning-free methods;
m. Combining the predictive spaces for the catalyst and the substrate built in stage *e* for each training catalyst - substrate system;
n. Building a mathematical equation which matches combined predictive space according to stage f and chemical behaviour in a given chemical process for each training catalyst - substrate system by using regression methods.

2. A computer-based method for predicting the chemical behaviour of a catalyst - substrate system in a chemical process, comprising:
i. Generating 3D quantum descriptors for a series of training catalyst - substrate systems by using a universal quantum mechanics based method;
j. Computing the electronic density and electrostatic potential for a series of 3D points inside the grid which encloses each training catalyst - substrate system;
k. Building a molecular isosurface for each training catalyst - substrate system from the electronic density values computed in stage b;
l. Computing an steric descriptor at each isosurface 3D point;
m. Building predictive spaces for the catalyst and the substrate comprised in each training catalyst - substrate system from the electrostatic potential values computed in stage b and the steric descriptors computed in stage d by using structural aligning-free methods;
n. Combining the predictive spaces for the catalyst and the substrate built in stage e for each training catalyst - substrate system;
o. Building a mathematical equation which matches combined predictive space according to stage f and chemical behaviour of each training catalyst - substrate system, by using regression methods;
p. Predicting the chemical behaviour of a catalyst - substrate system in a chemical process by using the mathematical equation built in stage g.

3. A computer-based method according to claim 2, wherein the chemical behaviour is selected from the group comprising absolute configuration induced, enantiomeric excess induced or the combination of both.

4. A computer-based method for designing catalysts with a chemical targeted behaviour in a chemical process, comprising:
k. Generating 3D quantum descriptors for a series of training catalyst - substrate systems by using a universal quantum mechanics based method;
l. Computing the electronic density and electrostatic potential for a series of 3D points inside the grid which encloses each training catalyst - substrate system;
m. Building a molecular isosurface for each training catalyst - substrate system from the electronic density values computed in stage b;
n. Computing an steric descriptor at each isosurface 3D point;
o. Building predictive spaces for the catalyst and the substrate comprised in each training catalyst - substrate system from the electrostatic potential values computed in stage b and the steric descriptors computed in stage d by using structural aligning-free methods;
p. Combining the predictive spaces for the catalyst and the substrate built in stage *e* for each training catalyst - substrate system;
q. Building a mathematical equation which matches combined predictive space according to stage *f* and chemical behaviour in a given chemical process for each training catalyst - substrate system by using regression methods;
r. Analysing the relative influence of the catalyst and the substrate of test catalyst - substrate systems on the chemical behaviour by using the mathematical equation built in stage *g* and the predictive spaces;
s. Evaluating the relative influence of each functional group of the catalyst and the substrate on the chemical behaviour of each test catalyst - substrate system;
t. Using the information extracted from the stages *h* and *i* for designing catalysts with a targeted chemical behaviour.

5. The computer-based process according to claim 4, wherein the chemical targeted behaviour is selected from the group comprising absolute configuration induced, enantiomeric excess induced or the combination of both.

6. The computer-based process according to any of claims 1 to 5 further comprising the validation of the mathematical equation by using test catalyst - substrate systems.

7. A computer-based method according to any of claims 1 to 6, wherein the universal quantum mechanics based method is the *Density Functional Theory.*

8. A computer-based method according to any of claims 1 to 7, wherein the catalyst is a metal catalyst which comprises a bidentate ligand.

9. A computer-based method according to any of claims 1 to 8, wherein the chemical process is selected from the group comprising alkylations, conjugated additions, aldol reactions and Mannich reactions.

10. A computer-based method according to claim 8 or 9, wherein the catalyst is a Zn amino-alcohol catalyst or a Zn amino-alcohol catalyst precursor and the chemical process is the ethylation of an aldehyde.
